Europäisches Patentamt

(19) European Patent Office

Office européen des brevets

(11) Publication number: **0 019 363**
A1

## (12) EUROPEAN PATENT APPLICATION

(21) Application number: 80301224.4

(22) Date of filing: 14.04.80

(51) Int. Cl.³: **C 07 C 15/16**
C 07 C 1/26

(30) Priority: 11.05.79 GB 7916348
12.07.79 GB 7924303

(43) Date of publication of application:
26.11.80 Bulletin 80/24

(84) Designated Contracting States:
BE CH DE FR GB IT LI NL SE

(71) Applicant: IMPERIAL CHEMICAL INDUSTRIES LIMITED
Imperial Chemical House Millbank
London SW1P 3JF(GB)

(72) Inventor: Breeze, Alan Geoffrey
Forest Pines
Norley Sandiway Northwich(GB)

(72) Inventor: Morley, John Oswald
79 Ochiltree
Dunblane Perthshire Scotland FK15 0D6(GB)

(74) Representative: Roberts, John Stanley et al,
IMPERIAL CHEMICAL INDUSTRIES LIMITED Legal
Department: Patents Thames House North Millbank
London SW1P 4QG(GB)

(54) Preparation of diphenylmethane derivatives.

(57) Preparation of a 2-methyldiphenylmethane by reaction of
2-chloromethyltoluene or benzyl chloride with benzene or a
xylene respectively in the liquid phase in the presence of
liquid hydrogen fluoride.

EP 0 019 363 A1

Croydon Printing Company Ltd.

PREPARATION OF DIPHENYLMETHANE DERIVATIVES

1.                          MD/DX 30782

This invention relates to the preparation of diphenylmethane derivatives and more particularly to the preparation of substituted derivatives of 2-methyl-diphenylmethane and substituted derivatives thereof.

The compound 2-methyl-diphenylmethane and substituted derivatives thereof are intermediates in the synthesis of anthraquinone and substituted derivatives thereof. The compound referred to herein as 2-methyl-diphenylmethane may alternatively be described as 2-benzyltoluene, ortho-benzyltoluene or 1-methyl-2-(phenylmethyl)-benzene.

It has previously been proposed to prepare 2-methyl-diphenylmethane by the reaction between 2-chloromethyltoluene (ortho-xylyl chloride) and benzene using as catalyst relatively large amounts either of boron trifluoride or of an inorganic acid (for example sulphuric acid). These known processes have the disadvantage of requiring long reaction periods, or expensive catalysts, or difficult separation of the organic product from a large amount of catalyst.

According to the present invention there is provided a process for the preparation of a 2-methyl-diphenylmethane which comprises reacting 2-chloromethyltoluene or benzyl chloride with benzene or a xylene respectively in the liquid phase in the presence of liquid hydrogen fluoride.

The proportion of hydrogen fluoride is preferably at least O.5 mole per mole of 2-chloromethyltoluene or benzyl chloride. It is however an advantage of the process of the present invention that unconsumed hydrogen fluoride may readily be recovered and accordingly it is convenient to employ at least 1 mole (for example from 1 to 25 moles) of hydrogen fluoride per mole of 2-chloromethyltoluene or of benzyl chloride. Thus the reaction may be carried out using hydrogen fluoride, in effect, as the reaction medium, the upper limit to the ratio of hydrogen fluoride to 2-chloromethyltoluene or to benzyl chloride being set only by economic considerations such as the size of the reactor and of the hydrogen fluoride recovery system. An inert liquid diluent may, however, be present if desired. If a diluent is used this is preferably a liquid with a boiling point such that it may readily be removed by distillation at the end of the reaction. Suitable diluents include alkanes; thus, for example, when benzene is a reactant a convenient diluent is n-hexane.

It is preferred to use at least 1 mole of benzene or xylene (for example from 2 to 10 moles) per mole of 2-chloromethyltoluene or of benzyl chloride respectively. A larger excess of benzene or xylene may be used if desired, the upper limit again being set by economic considerations

3.

The reaction may be carried out over a wide temperature range, for example in the range from 0°C to 100°C. The reaction may conveniently be carried out, for example, at substantially ambient temperature under conditions of reflux of the hydrogen fluoride. Superatmospheric pressure may be used if desired.

At the higher temperatures within the range referred to, some demethylation of the 2-methyl group may occur, the extent depending upon the reaction period and the ratio of the reactants employed. Accordingly, it is in general advisable that the reaction temperature should not exceed 80°C, the range 0°C to 50°C (especially from 0°C to 30°C) being preferred.

When the reactants are 2-chloromethyltoluene and benzene the main product under the preferred conditions is 2-methyl-diphenylmethane itself.

When the reactants are benzyl chloride and m-xylene (1,3-xylene), the main product under the preferred conditions is 2,4-dimethyldiphenylmethane.

When the reactants are benzyl chloride and p-xylene (1,4-xylene), the main product under the preferred conditions is 2,5-dimethyldiphenylmethane.

When the reactants are benzyl chloride and o-xylene (1,2-xylene), a mixture of products may be obtained, including 2,3-dimethyldiphenylmethane.

The reaction may be carried out either batchwise or continuously.

At the end of the reaction any hydrogen fluoride remaining may readily be recovered by standard techniques (for example by vacuum distillation or, in appropriate cases, by phase separation) and, if desired, recycled to the process. Any excess xylene or benzene may similarly be recovered and recycled. The 2-methyl-diphenylmethane (or substituted derivative thereof) produced may be separated and purified by conventional methods.

The invention is illustrated by the following examples in which parts are parts by weight.

EXAMPLE 1

Liquid hydrogen fluoride (8 parts) was added to a mixture of p-xylene (63.6 parts) and benzyl chloride (12.6 parts) contained in a pressure vessel. The reaction mixture thus contained 6 moles of p-xylene and 4 moles of HF per mole of benzyl chloride.

The reaction mixture was maintained at 20°C for 60 minutes. The hydrogen chloride evolved during the reaction was then vented off. The reaction product was added to an excess of cold water; the organic layer was separated off, washed free from acid and dried over calcium chloride. Vacuum distillation yielded 18.0 parts (92% theory) of 2,5-dimethyldiphenylmethane (analysis by gas-liquid chromatography and nuclear magnetic resonance).

EXAMPLE 2

Liquid hydrogen fluoride (8 parts) was added to a mixture of m-xylene (63.6 parts) and benzyl chloride (12.6 parts) contained in a pressure vessel. The reaction mixture thus contained 6 moles of m-xylene and 4 moles of HF per mole of benzyl chloride.

The reaction mixture was maintained at 20°C for 30 minutes. At the end of this period the product was added to an excess of cold water and separated as described in Example 1. Vacuum distillation yielded 14.9 parts of 2,4-dimethyldiphenylmethane (76% theory).

EXAMPLE 3

Liquid hydrogen fluoride (26 parts) was added to a mixture of benzene (64 parts) and 2-chloromethyltoluene (19 parts) contained in a pressure vessel. The reaction mixture thus contained 6.1 moles of benzene and 9.6 moles of HF per mole of 2-chloromethyltoluene.

The reaction mixture was maintained at 20°C for 30 minutes. The hydrogen chloride evolved during the reaction was then vented off. The reaction product was added to an excess of cold water; the organic layer was separated off, washed free from acid and dried over calcium chloride. Vacuum distillation yielded 16.1 parts (65% theory) of 2-methyl-diphenyl-methane (analysis by gas-liquid chromatography).

EXAMPLE 4

Liquid hydrogen fluoride (15 parts) was added to a mixture of benzene (47 parts) and 2-chloromethyltoluene (14 parts) contained in a pressure vessel. The reaction mixture thus contained 6.0 moles of benzene and 7.5 moles of HF per mole of 2-chloromethyltoluene.

The reaction mixture was maintained at 20°C for 1 hour. At the end of this period the product was added to an excess of cold water and separated as described in Example 1. Vacuum distillation yielded 14 parts of 2-methyl-diphenylmethane (78% theory).

EXAMPLE 5

The proportions of reactants were the same as in Example 2 but the mixture was heated at 75°C for 8 hours.

Under these conditions some demethylation occured and vacuum distillation yielded 15 parts of a product consisting of 40% by weight of 2-methyl-diphenylmethane and 60% by weight of diphenylmethane.

EXAMPLE 6

Liquid hydrogen fluoride (400 parts) was added to a mixture of benzene (390 parts), 2-chloromethyltoluene (140.5 parts) and n-hexane (39 parts) contained in a polythene reaction vessel provided with a condenser. The reaction mixture thus contained 5 moles of benzene and 20 moles of HF per mole of 2-chloromethyltoluene.

The reaction mixture was maintained at 8°C to 12°C for 1 hour, with stirring. At the end of this period the organic layer was separated from the hydrogen fluoride layer, washed with water and then with dilute sodium carbonate solution. Excess benzene, together with the n-hexane, was removed by distillation at atmospheric pressure. The residue was distilled under vacuum (3 mm Hg) to yield 149 parts of substantially pure 2-methyl-diphenylmethane (82% theory).

EXAMPLE 7

The procedure of Example 6 was repeated except that the initial reaction mixture consisted of liquid hydrogen fluoride (441 parts), benzene (780 parts), 2-chloromethyltoluene (140.5 parts) and n-hexane (78 parts). The mixture thus contained 10 moles of benzene and 21 moles of HF per mole of 2-chloromethyltoluene.

The reaction period at 8°C to 12°C was 22 minutes. The reaction products were treated as described in Example 6 to yield 155 parts of substantially pure 2-methyldiphenylmethane (85% theory).

CLAIMS

1   A process for the preparation of a 2-methyl-
    diphenylmethane characterised in that 2-chloromethyltoluene or benzyl chloride is reacted
    with benzene or a xylene respectively in the
    liquid phase in the presence of liquid hydrogen
    fluoride.

2   A process according to Claim 1 characterised
    in that the proportion of hydrogen fluoride is
    at least O.5 mole per mole of 2-chloromethyltoluene
    or benzyl chloride.

3   A process according to Claim 2 characterised
    in that the proportion of hydrogen fluoride is
    from 1 to 25 moles per mole of 2-chloromethyltoluene or benzyl chloride.

4   A process according to any of Claims 1 to 3
    characterised in that the proportion of
    benzene or xylene is at least 1 mole per mole
    of 2-chloromethyltoluene or benzyl chloride
    respectively.

5   A process according to any of the preceding
    claims characterised in that the reaction
    temperature is in the range from O°C to 5O°C.

6   A process according to any of the preceding
    claims characterised in that benzyl chloride
    is reacted with p-xylene or m-xylene.

TD/243/BO8/JSR/DT

## DOCUMENTS CONSIDERED TO BE RELEVANT

| Category | Citation of document with indication, where appropriate, of relevant passages | Relevant to claim |
|---|---|---|
| | GB - A - 1 488 185 (BASF)<br>* claim 1; page 5, lines 57, 61; examples * <br><br>-- <br><br>GB - A - 1 467 386 (BASF)<br>* claims 1, 10; examples *<br><br>---- | 1,4<br><br><br><br>1,4 |

**CLASSIFICATION OF THE APPLICATION (Int. Cl.3)**

C 07 C    15/16
C 07 C    1/26

**TECHNICAL FIELDS SEARCHED (Int.Cl.3)**

C 07 C    1/26
C 07 C    15/16

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 21-08-1980 | KNAACK |

EPO Form 1503.1    06.78